# FASCICULE DE BREVET EUROPEEN

(11) **EP 1 907 535 B1**
(45) Date de publication et mention de la délivrance du brevet: **12.09.2012**
(21) Numéro de dépôt: 06778633.5
(22) Date de dépôt: 21.06.2006
(51) Int. Cl.: C12N 7/00, C12N 15/86, A61K 39/12, C07K 14/18, C12N 15/113

(54) **CONSTRUCTION D'ADNC D'ALPHAVIRUS DE SALMONIDES**
CDNA-KONSTRUKT VON SALMONIDAE-ALPHAVIRUS
CDNA CONSTRUCT OF SALMONIDAE ALPHAVIRUS

(30) Priorité: 21.06.2005 FR 0506275
(43) Date de publication de la demande: 09.04.2008
(73) Titulaire: INSTITUT NATIONAL DE LA RECHERCHE AGRONOMIQUE (INRA), 75007 Paris Cedex 07 (FR)
(72) Inventeur: LEBERRE, Monique, F-78180 Montigny-le Bretonneux (FR); MORIETTE, Coralie, F-75013 Paris (FR); BREMONT, Michel, F-94600 Choisy Le Roi (FR)
(74) Mandataire: Keus, Jacobus Albertus Ronald
(86) Numéro de dépôt international: PCT/FR2006/001405
(87) Numéro de publication internationale: WO 2006/136704

(56) Documents cités:
- US-A1- 2004 258 707
- US-A1- 2004 258 707
- SMERDOU C ET AL: "Non-viral amplification systems for gene transfer: vectors based on alphaviruses." CURRENT OPINION IN MOLECULAR THERAPEUTICS. APR 1999, vol. 1, no. 2, avril 1999 (1999-04), pages 244-251, XP009062410 ISSN: 1464-8431
- MCCORMICK CHRISTOPHER J ET AL: "Introduction of replication-competent hepatitis C virus transcripts using a tetracycline-regulable baculovirus delivery system." JOURNAL OF GENERAL VIROLOGY, vol. 85, no. 2, février 2004 (2004-02), pages 429-439, XP002369434 ISSN: 0022-1317
- FROLOV I ET AL: "ALPHAVIRUS-BASED EXPRESSION VECTORS: STRATEGIES AND APPLICATIONS" PROCEEDINGS OF THE NATIONAL ACADEMY OF SCIENCES OF USA, NATIONAL ACADEMY OF SCIENCE, WASHINGTON, DC, US, vol. 93, no. 21, 1996, pages 11371-11377, XP000910193 ISSN: 0027-8424 cité dans la demande
- RUFFNER D E ET AL: "Sequence requirements of the hammerhead RNA self-cleavage reaction." BIOCHEMISTRY. 27 NOV 1990, vol. 29, no. 47, 27 novembre 1990 (1990-11-27), pages 10695-10702, XP002369435 ISSN: 0006-2960 cité dans la demande
- SHIPPY RICHARD ET AL: "The hairpin ribozyme: Discovery, mechanism, and development for gene therapy" MOLECULAR BIOTECHNOLOGY, vol. 12, no. 1, août 1999 (1999-08), pages 117-129, XP009062409 ISSN: 1073-6085
- WESTON JONATHAN ET AL: "Comparison of two aquatic alphaviruses, salmon pancreas disease virus and sleeping disease virus, by using genome sequence analysis, monoclonal reactivity, and cross-infection" JOURNAL OF VIROLOGY, vol. 76, no. 12, juin 2002 (2002-06), pages 6155-6163, XP002369436 ISSN: 0022-538X
- VILLOING STEPHANE ET AL: "Rainbow trout sleeping disease virus is an atypical alphavirus" JOURNAL OF VIROLOGY, vol. 74, no. 1, janvier 2000 (2000-01), pages 173-183, XP002369437 ISSN: 0022-538X
- MORIETTE CORALIE ET AL: "Recovery of a recombinant Salmonid alphavirus fully attenuated and protective for rainbow trout" JOURNAL OF VIROLOGY, vol. 80, no. 8, avril 2006 (2006-04), pages 4088-4098, XP009076629 ISSN: 0022-538X
- SARDYKO S V: "ADAPTIVE QUANTIZATION OF TV IMAGE SEGMENTS", TEKHNIKA KINO I TELEVIDENIYA, ISKUSSTVO, MOSKVA, RU, vol. 1, no. 10, 1 October 1988 (1988-10-01), pages 24-26, XP009062410, ISSN: 0040-2249
- MCCORMICK C J ET AL: "Introduction of replication-competent hepatitis C virus transcripts using a tetracycline-regulable baculovirus delivery system", JOURNAL OF GENERAL VIROLOGY, SOCIETY FOR GENERAL MICROBIOLOGY, SPENCERS WOOD, GB, vol. 85, no. 2, 1 February 2004 (2004-02-01), pages 429-439, XP002369434, ISSN: 0022-1317
- FROLOV I ET AL: "ALPHAVIRUS-BASED EXPRESSION VECTORS: STRATEGIES AND APPLICATIONS", PROCEEDINGS OF THE NATIONAL ACADEMY OF SCIENCES OF USA, NATIONAL ACADEMY OF SCIENCE, WASHINGTON, DC, US, vol. 93, no. 21, 1 January 1996 (1996-01-01), pages 11371-11377, XP000910193, ISSN: 0027-8424
- RUFFNER D E ET AL: "Sequence requirements of the hammerhead RNA self-cleavage reaction", BIOCHEMISTRY, AMERICAN CHEMICAL SOCIETY, EASTON, PA.; US, vol. 29, no. 47, 27 November 1990 (1990-11-27), pages 10695-10702, XP002369435, ISSN: 0006-2960
- SHIPPY R ET AL: "THE HAIRPIN RIBOZYME: DISCOVERY, MECHANISM, AND DEVELOPMENT FOR GENE THERAPY", MOLECULAR BIOTECHNOLOGY, HUMANA PRESS, INC, US, vol. 12, no. 1, 1 August 1999 (1999-08-01) , pages 117-129, XP009062409, ISSN: 1073-6085
- WESTON J ET AL: "Comparison of two aquatic alphaviruses, salmon pancreas disease virus and sleeping disease virus, by using genome sequence analysis, monoclonal reactivity, and cross-infection", JOURNAL OF VIROLOGY, THE AMERICAN SOCIETY FOR MICROBIOLOGY, US, vol. 76, no. 12, 1 June 2002 (2002-06-01), pages 6155-6163, XP002369436, ISSN: 0022-538X
- VILLOING S ET AL: "Rainbow trout sleeping disease virus is an atypical alphavirus", JOURNAL OF VIROLOGY, THE AMERICAN SOCIETY FOR MICROBIOLOGY, US, vol. 74, no. 1, 1 January 2000 (2000-01-01), pages 173-183, XP002369437, ISSN: 0022-538X
- MORIETTE CORALIE ET AL: "RECOVERY OF A RECOMBINANT SALMONID ALPHAVIRUS FULLY ATTENUATED AND PROTECTIVE FOR RAINBOW TROUT", JOURNAL OF VIROLOGY, THE AMERICAN SOCIETY FOR MICROBIOLOGY, US, vol. 80, no. 8, 1 April 2006 (2006-04-01), pages 4089-4098, XP009076629, ISSN: 0022-538X

### SEQUENCE LISTING

<110> INSTITUT NATIONAL DE LA RECHERCHE AGRONOMIQUE LEBERRE, Monique MORIETTE, Coralie BREMONT, Michel
<120> Construction d'ADNc d'alphavirus de salmonidés.
<130> MJP/mad-F539/127-WO
<150> FR 05 06275
   <151> 2005-06-21
<160> 24
<170> PatentIn version 3.3
<210> 1
   <211> 42
   <212> DNA
   <213> Artificial
<220>
   <223> Amorce PCR
<400> 1
   ccgaattcgt taaatccaaa agcatacata tatcaatgat gc 42
<210> 2
   <211> 33
   <212> DNA
   <213> Artificial
<220>
   <223> Amorce PCR
<400> 2
   cccggggcgg ccccaaggtc gagaactgag ttg 33
<210> 3
   <211> 35
   <212> DNA
   <213> Artificial
<220>
   <223> Amorce PCR
<400> 3
   ccccgggagg agtgaccgac tactgcgtga agaag 35
<210> 4
   <211> 31
   <212> DNA
   <213> Artificial
<220>
   <223> Amorce PCR
<400> 4
   ggtctagagt atgatgcaga aaatattaag g 31
<210> 5
   <211> 35
   <212> DNA
   <213> Artificial
<220>
   <223> Amorce PCR
<400> 5
   cctctagacc aaccatgttt cccatgcaat tcacc 35
<210> 6
   <211> 42
   <212> DNA
   <213> Artificial
<220>
   <223> Amorce PCR
<400> 6
   ccgcggccgc attgaaaatt ttaaaaacca atagatgact ca 42
<210> 7
   <211> 79
   <212> DNA
   <213> Artificial
<220>
   <223> Amorce PCR
<400> 7
   ggatcctgga tttatcctga tgagtccgtg aggacgaaac tataggaaag gaattcctat 60
   agtcgataaa tccaaaagc 79
<210> 8
   <211> 80
   <212> DNA
   <213> Artificial
<220>
   <223> Amorce PCR
<400> 8
   gccggcggaa gggttagctg tgagattttg catcattgat atatgtatgc ttttggattt 60
   atcgactata ggaattcctt 80
<210> 9
   <211> 25
   <212> DNA
   <213> Artificial
<220>
   <223> Amorce PCR
<400> 9
   cctcgtcagc gggacccata atgcc 25
<210> 10
   <211> 30
   <212> DNA
   <213> Artificial
<220>
   <223> Amorce PCR
<400> 10
   ccgctgagcg gttggttgag agtatgatgc 30
<210> 11
   <211> 32
   <212> DNA
   <213> Artificial
<220>
   <223> Amorce PCR
<400> 11
   ccaaccgctg agcatggtga gcaagggcga gg 32
<210> 12
   <211> 47
   <212> DNA
   <213> Artificial
<220>
   <223> Amorce PCR
<400> 12
   gtggctaacg gcaggtgatt cacgcttaag ctcgagatct gagtccg 47
<210> 13
   <211> 45
   <212> DNA
   <213> Artificial
<220>
   <223> Amorce PCR
<400> 13
   gcgtgaatca cctgccgtta gccacaatgg cgatggccac gctcg 45
<210> 14
   <211> 32
   <212> DNA
   <213> Artificial
<220>
   <223> Amorce PCR
<400> 14
   ccatgctgag cggttggttg agagtatgat gc 32
<210> 15
   <211> 25
   <212> DNA
   <213> Artificial
<220>
   <223> Amorce PCR
<400> 15
   ggcggcttcc tgttactcga cacgg 25'
<210> 16
   <211> 39
   <212> DNA
   <213> Artificial
<220>
   <223> Amorce PCR
<400> 16
   atcgatgaac gatatcggcc gccgctacac gctatggcg 39
<210> 17
   <211> 35
   <212> DNA
   <213> Artificial
<220>
   <223> Amorce PCR
<400> 17
   ccggaatgct agcttaagct cgagatctga gtccg 35
<210> 18
   <211> 34
   <212> DNA
   <213> Artificial
<220>
   <223> Amorce PCR
<400> 18
   cgagcttaag ctagcattcc ggtatacaaa tcgc 34
<210> 19
   <211> 38
   <212> DNA
   <213> Artificial
<220>
   <223> Amorce PCR
<400> 19
   ggctaggtcg gcggccgcaa aaaacccctc aagacccg 38
<210> 20
   <211> 23
   <212> DNA
   <213> Artificial
<220>
   <223> Amorce PCR
<400> 20
   ccgccgagtc gctccagttg gcg 23
<210> 21
   <211> 25
   <212> DNA
   <213> Artificial
<220>
   <223> Amorce PCR
<400> 21
   cgggttctcc aggacgtcct tcaag 25
<210> 22
   <211> 28
   <212> DNA
   <213> Artificial
<220>
   <223> Amorce PCR
<400> 22
   ggcggcggca tggtcgttgg acgaccgg 28
<210> 23
   <211> 28
   <212> DNA
   <213> Artificial
<220>
   <223> Amorce PCR
<400> 23
   ccttcagcat agtcatggcc ttctttgg 28
<210> 24
   <211> 25
   <212> DNA
   <213> Artificial
<220>
   <223> Amorce PCR
<400> 24
   ttaagctcga gatctgagtc cggac 25

## Description

La présente invention est relative à l'obtention d'ADNc infectieux d'alphavirus de salmonidés, et aux utilisations de ces ADNc.

Les alphavirus sont des virus enveloppés à ARN positif de la famille des *Togaviridae.*

On connaît actuellement deux représentants des alphavirus de salmonidés : le virus de la maladie du sommeil (VMS), pathogène de la truite, et le virus de la maladie du pancréas (VMP) pathogène du saumon. Ces deux virus, qui sont très proches génétiquement, ont été rattachés à la famille des alphavirus, sur la base de la similitude de l'organisation de leur génome avec celui des alphavirus connus ; cependant, leur séquence nucléotidique, ainsi que la séquence polypeptidique qui en est déduite ne présentent qu'un degré d'homologie faible avec les autres alphavirus (VILLOING et al., J. Virol. 74, 173-183, 2000 ; WESTON et al., Virology, 256, 188-195, 1999 ; WESTON et al., J. Virol 76, 6155-63, 2002) ; ils sont donc considérés comme représentant un sous groupe d'alphavirus, distinct des alphavirus de mammifères.

Le génome des alphavirus code pour 2 polyprotéines : la portion 5' de la séquence codante, qui représente environ les deux tiers de celle-ci, code pour une polyprotéine qui après clivage protéolytique, donne les protéines non-structurales (nsP) nsP1, nsP2, nsP3 et nsP4, impliquées dans la réplication du virus ; le tiers terminal du génome code pour une polyprotéine dont le clivage protéolytique produit les protéines structurales (Struct) : la protéine de capside (C), et 2 glycoprotéines d'enveloppe (E3-E2 et 6K-E1). Les régions codantes pour les nsP et les Struct sont séparés par une région non codante appelé région de jonction (J). Deux régions non codantes sont également présentes en aux extrémités 5' et 3' du génome. Ce génome possède en outre une coiffe à l'extrémité 5' et une queue polyA à l'extrémité 3' (STRAUSS et STRAUSS, Microbiol. Rev., 58, 491-562, 1994).

Chez les alphavirus de salmonidés, cette organisation génomique est conservée: les différences principales avec le génome des autres alphavirus, sont, outre la faible homologie des séquences codantes, la taille des régions 5' et 3' non-codantes, qui sont plus courtes chez les alphavirus de salmonidés.

Les pathologies causées par le VMS et le VMP constituent actuellement un problème croissant pour la salmoniculture. Ces virus deviennent maintenant endémiques en Europe et ont fait leur apparition sur le continent Nord américain.

Le mode de protection des salmonidés contre ces agents viraux réside dans l'utilisation de vaccins. La vaccination peut être effectuée de différentes manières, selon l'espèce et l'âge du poisson à vacciner. Elle peut s'effectuer par exemple par balnéation. Cette voie de vaccination, qui est très efficace et peu coûteuse, est la méthode de choix pour la vaccination par virus vivants atténués. La vaccination peut également être effectuée par injection de virus vivants atténués, ou de virus inactivés. Bien que plus coûteuse que la vaccination par balnéation, cette méthode est utilisable pour la vaccination de poissons de 10 grammes ou plus, et convient ainsi notamment pour la vaccination d'espèces comme le saumon ou la truite de mer. Avantageusement, on peut également combiner une primo-vaccination par balnéation en présence de virus atténués vivants, et une vaccination de rappel par injection de virus vivants atténués, ou de virus inactivés.

La manipulation du génome des virus à ARN, notamment pour générer des souches atténuées nécessite de disposer d'un système d'ADNc infectieux, c'est-à-dire d'une copie complète ADN du génome ARN de ces virus (environ 12000 bases), capable d'être transcrite dans une cellule-hôte pour générer un ARN viral pouvant se répliquer dans cette cellule.

Classiquement, pour produire un ADNc infectieux pour les alphavirus, on clone dans un plasmide l'ADNc du génome viral complet, fusionné à l'extrémité 5' à une séquence promotrice SP6 ou T7. Cette construction plasmidique est clivée à l'extrémité 3' par une enzyme de restriction, puis utilisée dans un système de transcription *in vitro* pour la synthèse d'un ARN génomique par l'ARN polymérase SP6 ou T7. Cet ARN est transfecté dans des cellules sensibles au virus considéré. Après quelques jours, du virus néoformé est relargué dans le surnageant de culture. On a ainsi pu obtenir des ADNc infectieux pour un grand nombre d'alphavirus, par exemple pour le SV (Sindbis virus ; RICE et al., J. Virol, 61, 3809-3819, 1987), le SFV (Semliki Forest Virus ; LILJESTROM et al., J. Virol, 65, 4107-4113, 1991), le virus VEE (Venezuelan equine encephalitis ; DAVIS et al., Virology, 171, 189-204, 1989, le virus EEE (eastern equine encephalitis ; SCHOEPP et al., Virology, 302, 299-309, 2002).

Cependant, dans le cas des alphavirus de salmonidés, les tentatives effectuées pour générer un ADNc infectieux en utilisant cette approche ont échoué.

Les inventeurs ont découvert que ce problème pouvait être résolu en introduisant une séquence supplémentaire aléatoire, jouant le rôle d'espaceur, entre le promoteur SP6 ou T7 et le début du génome viral. L'addition de cette séquence permet de générer *in vitro* un ARN génomique qui par transfection dans des cellules de poissons permet la synthèse des protéines non structurales.

Pour générer un ARN infectieux, les Inventeurs ont remplacé la séquence aléatoire jouant le rôle d'espaceur par une séquence de ribozyme en tête de marteau. Ils ont observé que l'ARN néo-synthétisé à partir de cette construction était clivé de manière précise au premier nucléotide de l'ARN génomique de l'alphavirus. L'ARN ainsi obtenu a la capacité de synthétiser les protéines nsP, d'être répliqué, de permettre la synthèse d'un ARN subgénomique codant les protéines structurales, et enfin d'être encapsidé pour générer des néoparticules virales infectieuses.

Les Inventeurs ont en outre utilisé cette construction pour insérer une séquence hétérologue, sous contrôle du promoteur subgénomique du VMS. Ils ont observé que cette séquence était exprimée dans les cellules infectées par la construction, et que le génome viral comprenant cette séquence pouvait être encapsidé normalement dans les particules virales.

La présente invention a pour objet un ADN recombinant dérivé du génome d'un alphavirus de salmonidé, et comprenant :
- un promoteur de transcription, et en aval dudit promoteur et sous contrôle transcriptionnel de celui-ci ;
- une séquence d'espacement d'au moins 5 nucléotides, de préférence de 10 à 100 nucléotides ;
- l'ADNc de l'ARN génomique d'un alphavirus de salmonidé.

Le promoteur de transcription peut être n'importe quel promoteur reconnu par une ARN polymérase exprimée dans la cellule-hôte. Il peut s'agir par exemple d'un promoteur de bactériophage, comme le promoteur T7, le promoteur T3, ou le promoteur SP6 ; dans ce cas, l'ADN recombinant conforme à l'invention doit être utilisé en association avec une construction exprimant une ARN polymérase reconnaissant ce promoteur.

On peut utiliser également un promoteur reconnu par une ARN polymérase endogène de la cellule-hôte, en particulier par l'ARN polymérase II. Il peut s'agir d'un promoteur viral, par exemple l'un de ceux habituellement utilisés pour l'expression de gènes hétérologues dans des cellules de mammifères, tels que le promoteur CMV (cytomégalovirus), le promoteur RSV (Rous Sarcoma Virus), le promoteur précoce de SV40, le promoteur MoMLV (Moloney Murine Leukemia Virus), etc. ; il peut s'agir aussi d'un promoteur eucaryote, par exemple d'un promoteur de poisson, tel que celui décrit par ALONSO et al. (Vaccine, 21, 1591-1600,2003)

La fonction de la séquence d'espacement est d'éloigner le promoteur du début du génome de l'alphavirus ; sa séquence n'est donc pas critique pour la mise en oeuvre de la présente invention. En ce qui concerne sa longueur, les Inventeurs ont observé qu'une séquence de 6 nucléotides pouvait conférer un espacement suffisant. Généralement on préférera utiliser une séquence plus longue, d'environ 10 à 100 nucléotides, et préférentiellement de 50 à 100 nucléotides.

Très avantageusement, on utilisera une séquence d'espacement permettant l'insertion, à l'extrémité 5' de l'ARN génomique, d'un ribozyme en tête de marteau.

Les ribozymes en tête de marteau sont de petits ribozymes (généralement d'environ 40 à 80 nucléotides) qui ont en commun une structure secondaire formée de 3 hélices dont la taille et la séquence peuvent varier, reliées par un noyau central conservé qui est essentiel pour l'activité catalytique (RUFFNER et al., Biochemistry, 29, 10695-10702, 1990).

La séquence permettant l'insertion, d'un ribozyme en tête de marteau à l'extrémité 5' de l'ARN génomique d'un alphavirus de salmonidé est définie par la formule générale (I) ci-après :

5' X₁CTGANGARX₂B₂X'₂YGAAAX₃B₃X'₃TH 3' (I)

dans laquelle, A, T, G, et C ont leur signification usuelle ; H représente C, T, ou A ; Y représente A ou G ; R représente C ou T ; N représente A, T, G, ou C ; X₁ représente un oligonucléotide d'au moins 3 nucléotides, de préférence de 6 à 10 nucléotides, de séquence complémentaire de celle de l'extrémité 5' du génome dudit alphavirus ; X₂ représente un oligonucléotide d'au moins 3 nucléotides, de préférence de 3 à 5 nucléotides, de séquence quelconque ; B₂ représente un oligonucléotide de 4 ou 5 nucléotides, de séquence quelconque ; X'₂ représente un oligonucléotide complémentaire de X₂; X₃ représente un oligonucléotide d'au moins 2 nucléotides, de préférence de 6 à 10 nucléotides, de séquence quelconque ; B₃ représente un oligonucléotide de 4 ou 5 nucléotides, de séquence quelconque ; X'₃ représente un oligonucléotide complémentaire de X₃.

Afin d'assurer la terminaison correcte de l'ARN d'alphavirus dans le cas où l'on utilise un promoteur de bactériophage, l'ADN recombinant conforme à l'invention comprendra en outre, de manière classique, le terminateur de transcription qui correspond au promoteur utilisé. Dans le cas où l'on utilise un promoteur reconnu par une ARN polymérase endogène de la cellule hôte, on utilise une queue polyA, fusionnée à l'extrémité 3' du génome viral.

Les ADN recombinants conformes à l'invention peuvent être facilement construits à partir de l'ADNc obtenu par rétrotranscription de l'ARN génomique de l'alphavirus de salmonidé choisi. On peut, si on le souhaite, effectuer différentes modifications dans cet ADNc, selon l'utilisation envisagée pour l'ADN recombinant conforme à l'invention. Il peut s'agir par exemple de l'introduction d'un ou plusieurs sites de restriction, de la délétion de portions du génome viral, notamment de portions non nécessaires à sa réplication (par exemple tout ou partie de la région codant pour les protéines structurales), de la duplication de séquences virales, de l'insertion de séquences hétérologues, etc. Il peut s'agir également de mutations dont on souhaite tester les effets sur les propriétés de ces alphavirus, par exemple sur leur pouvoir infectieux, leur pathogénicité, ou leur antigénicité.

Les expressions « ADNc de l'ARN génomique d'un alphavirus de salmonidé » ou « ADNc d'un alphavirus de salmonidé », utilisées ici, doivent être interprétées comme englobant aussi bien l'ADNc obtenu par rétrotranscription de l'ARN génomique dudit l'alphavirus, que l'ADNc modifié comme indiqué ci-dessus.

La présente invention permet ainsi d'effectuer la manipulation du génome d'alphavirus de salmonidés, en vue de différentes applications, et de produire en grande quantité, et de manière reproductible, les alphavirus de salmonidés ainsi obtenus.

La présente invention permet notamment de construire à partir d'alphavirus de salmonidés, des vecteurs d'expression de structure similaire à ceux déjà construits à partir d'autres alphavirus (pour revue, cf. par exemple FROLOV et al., Proc Natl. Acad. Sci. USA, 93, 11371-11377, 1996). Ces vecteurs d'expression peuvent être de deux types principaux :
- des vecteurs capables de se répliquer, d'exprimer la séquence hétérologue qui y est insérée, et de s'encapsider pour produire de nouvelles particules virales. Ces vecteurs sont généralement obtenus à partir du génome complet d'un alphavirus en insérant dans celui-ci la séquence hétérologue d'intérêt placée sous contrôle d'une copie du promoteur subgénomique ;
- des vecteurs capables de se répliquer et d'exprimer la séquence hétérologue qui y est insérée, mais incapables de produire de nouvelles particules virales. Ces vecteurs sont généralement obtenus en remplaçant la région du génome de l'alphavirus codant pour les protéines de structure par la séquence hétérologue d'intérêt. L'encapsidation du virus ne peut s'effectuer que si les protéines de structure sont fournies en *trans* dans les cellules hôtes, par exemple du fait de réintroduction dans celles-ci de vecteurs auxiliaires exprimant ces protéines, ou du fait de l'utilisation, en tant que cellules hôtes, de lignées cellulaires transformées de façon stable par des cassettes d'expression exprimant ces protéines.

Des ADNs recombinants conformes à l'invention peuvent ainsi être utilisés pour l'expression d'une séquence hétérologue d'intérêt sous contrôle d'un promoteur subgénomique d'un alphavirus de salmonidé. Dans ce cas, l'insert d'ADNc d'alphavirus de salmonidé contient une ou plusieurs cassette(s) d'expression(s) dont chacune contient : un exemplaire dudit promoteur subgénomique, et, en aval dudit promoteur subgénomiqué et sous contrôle transcriptionnel de celui-ci, une séquence hétérologue que l'on souhaite exprimer, ou un site de clonage permettant l'insertion de cette séquence.

Le promoteur subgénomique des alphavirus est reconnu par le complexe nsP, et contrôle la transcription des gènes codant pour les protéines de structure Chez les alphavirus de salmonidés, ce promoteur est situé dans la région du génome comprenant la fin de la séquence codant pour nsp4, et la région de jonction (dans le cas du VMS, ce promoteur est situé dans la région correspondant aux nucléotides 7686-7846 du génome, comprenant les 124 derniers nucléotides de la séquence codant pour nsp4, et la région de jonction).

La séquence hétérologue peut être une séquence codant pour une protéine d'intérêt, ou bien une séquence transcrite en un ARN d'intérêt, par exemple un ARN antisens ou un ARN interférent.

La présente invention a également pour objet une méthode pour obtenir un ARN d'un alphavirus de salmonidé, caractérisée en ce qu'elle comprend l'introduction d'une construction conforme à l'invention dans une cellule-hôte appropriée, et la culture de ladite cellule-hôte.

Les cellules-hôtes utilisables dans le cadre de la présente invention sont de préférence des cellules de poisson ; à titre d'exemples non limitatifs on citera les cellules BF-2 (ATCC CCL-91), CHSE214 (ATCC CCL55) ou RTG-2 (ATCC CRL-1681). Si nécessaire, ces cellules sont transformées, de façon transitoire ou de façon stable, par une construction exprimant une ARN polymérase reconnaissant le promoteur utilisé pour la construction de l'ADN recombinant conforme à l'invention.

La présente invention a aussi pour objet une méthode pour obtenir un réplicon d'ARN d'alphavirus de salmonidé, caractérisé en ce qu'elle comprend l'introduction dans une cellule-hôte appropriée, d'un ADN recombinant conforme à l'invention dans laquelle la séquence d'espacement est une séquence de ribozyme en tête de marteau définie par la formule générale (I), et la culture de ladite cellule-hôte.

On définit ici par : "réplicon d'ARN" une molécule d'ARN capable de se répliquer de manière autonome dans une cellule-hôte.

Pour la production d'alphavirus recombinants, il est nécessaire que l'ensemble des protéines structurales nécessaires à l'encapsidation soit également exprimé dans la cellule-hôte. Cette expression peut s'effectuer en cis (l'ensemble de l'information génétique nécessaire à l'expression de ces protéines est portée par le réplicon d'ARN d'alphavirus), ou bien en *trans* (le réplicon d'ARN d'alphavirus ne porte pas l'ensemble de l'information génétique nécessaire à l'expression de ces protéines, et l'information génétique manquante est fournie par la cellule-hôte).

Si l'ADN recombinant conforme à l'invention contient l'ensemble de l'information génétique nécessaire à l'encapsidation, le réplicon d'ARN produit peut s'encapsider dans la cellule-hôte pour produire des alphavirus de salmonidés recombinants, capables de pénétrer dans d'autres cellules, de répliquer leur génome et de s'encapsider dans celles-ci de manière autonome. De tels virus sont définis ici comme « virus infectieux ».

Si l'ADN recombinant conforme à l'invention ne contient pas l'ensemble de l'information génétique nécessaire à l'encapsidation (notamment si elle est dépourvue de tout ou partie de la région codant pour les protéines structurales), le réplicon d'ARN produit ne peut pas s'encapsider dans la cellule-hôte, à moins que celle-ci ne fournisse en *trans* l'information génétique permettant de complémenter la fonction d'encapsidation déficiente (par exemple si elle est transformée, de façon transitoire ou de façon stable, par une construction exprimant les protéines de structure manquantes). Dans ce dernier cas, des alphavirus de salmonidés recombinants peuvent être produits dans cette cellule-hôte. Ils sont capables de pénétrer dans d'autres cellules et de répliquer leur génome dans celles-ci, mais ne pourront pas s'y encapsider que si, comme la cellule-hôte initiale, elles peuvent complémenter en *trans* la fonction d'encapsidation déficiente. De tels virus sont définis ici comme « virus abortif ».

La présente invention a également pour objet les transcrits, ainsi que les réplicons d'ARN d'alphavirus de salmonidés atténués et les alphavirus de salmonidés atténués recombinants, infectieux ou abortif, susceptibles d'être obtenus, comme décrit ci-dessus, à partir des ADNs recombinants conformes à l'invention.

La présente invention englobe plus particulièrement les réplicons d'ARN, et les alphavirus de salmonidés recombinants susceptibles d'être obtenus à partir d'ADNs recombinants conformes à l'invention dans lesquels une ou plusieurs modifications ont été introduites, comme indiqué ci-dessus, dans l'ADNc de l'alphavirus.

Les ADNs recombinants, les réplicons d'ARN, et les alphavirus de salmonidés recombinants d'expression conformes à l'invention peuvent être utilisés pour l'obtention de vaccins, par exemple pour l'obtention de souches vaccinales d'alphavirus de salmonidés atténuées ou inactivées.

La présente invention a également pour objet les vaccins comprenant un ADN recombinant, un réplicon d'ARN, ou un alphavirus de salmonidé recombinant conforme à l'invention, ou susceptibles d'être obtenus à partir de ceux-ci. Ces vaccins sont utilisables notamment pour protéger des salmonidés contre des infections à alphavirus.

La présente invention sera mieux comprise à l'aide du complément de description qui va suivre, qui se réfère à des exemples non-limitatifs illustrant l'obtention d'un ADN recombinant, de réplicons d'ARN, et de virus recombinants conformes à l'invention, à partir du VMS.

### EXEMPLES

### Virus et cellules

Les virus utilisés dans les exemples qui suivent sont dérivés de la souche S49P de VMS, précédemment décrite (CASTRIC et al., Bulletin of the European Association of Fish Pathologists, 17, 27-30, 1997).

Ces virus sont propagés sur des cultures en monocouche de cellules BF-2, cultivées à 10°C en milieu essentiel minimum de Eagle (EMEM, Sigma FRANCE) tamponné à pH 7.4 avec du Tris-HCl et supplémenté avec 10% de sérum de veau foetal. Afin d'obtenir un meilleur rendement lors des transfections, les cellules BF-2 utilisées sont issues de sous-clones sélectionnés sur la base de leur aptitude à être transfectés efficacement.

Cette sélection a été effectuée comme suit :

Des cellules BF-2 ont été mises en culture dans une plaque à 96 puits, à raison d'une cellule par puits. Au bout d'un mois 24 des clones ainsi obtenus ont été choisis au hasard, et amplifiés dans 2 plaques à 12 puits. Chacun de ces clones a été transfecté par un plasmide-test (pcDNA3-G), obtenu par insertion de la séquence codant pour la glycoprotéine G du virus VHSV (Viral Haemorrhagic Septicaemia Virus), dans le vecteur pcDNA3 (InVitrogen), en aval du promoteur CMV et du promoteur T7. L'efficacité de la transfection a été déterminée en évaluant le niveau d'expression de la glycoprotéine G sous contrôle du promoteur CMV, par immunofluorescence, à l'aide utilisant un anticorps dirigé contre cette protéine.

Les onze clones dans lesquels la fluorescence était la plus forte ont été sélectionnés, et amplifiés. Chacun de ces clones a été à nouveau testé, comme indiqué ci-dessus, pour sa capacité à être transfecté par pcDNA3-G, mais, cette fois-ci, après infection préalable avec vTF7-3 (FUERST et al., Proc. Natl. Acad. Sci. USA 83, 8122-8126, 1986), et en évaluant le niveau d'expression de la glycoprotéine G sous contrôle du promoteur T7. Finalement, 5 clones ont été choisis pour leur capacité à être infectés avec vTF7-3, combinée à leur aptitude à être transfectés efficacement.

### Amorces d'amplification :

Les séquences des amorces utilisées dans les exemples qui suivent sont indiquées dans le Tableau I ci-après.

**TABLEAU I**

| Amorce | Sequence (5'-3')* | Site de restriction | SEQ ID NO: |
|---|---|---|---|
| P1 | CCGAATTCGTTAAATCCAAAAGCATACATATATCAATGATGC | *EcoRI* | 1 |
| P2 | CCCGGGGCGGCCCCAAGGTCGAGAACTGAGTTG, | *SmaI* | 2 |
| P3 | CCCCGGGAGGAGTGACCGACTACTGCGTGAAGAAG | *SmaI* | 3 |
| P4 | GGTCTAGAGTATGATGCAGAAAATATTAAGG | *XbaI* | 4 |
| P5 | CCTCTAGACCAACCATGTTTCCCATGCAATTCACC | *XbaI* | 5 |
| P6 | CCGCGGCCGCATTGAAAATTTTAAAAACCAATAGATGACTCA | *NotI* | 6 |
| 5'RIBO | GGATCCTGGATTTATCCTGATGAGTCCGTGAGGACGAAACTATAGGAAAGGAATTCCTATAGTCGATAAATCCAAAAGC | *BamHI* | 7 |
| 3'RIBO | GCCGGCGGAAGGGTTAGCTGTGAGATTTTGCATCATTGATATATGTATGCTTTTGGATTTATCGACTATAGGAATTCCTT | *NaeI* | 8 |
| 5' SanDI | CCTCGTCAGCGGGACCCATAATGCC | *SanDI* | 9 |
| 3'?XbaIBlpI | CCGCTGAGCGGTTGGTTGAGAGTATGATGC | *BlpI* | 10 |
| 5'GFP | CCAACCGCTGAGCATGGTGAGCAAGGGCGAGG | *BlpI* | 11 |
| 3'GFP | *GTGGCTAACGGCAGGTGATTCACGC*TTAAGCTCGAGATCTGAGTCCG | - | 12 |
| 5'nsp4 | GCGTGAATCACCTGCCGTTAGCCACAATGGCGATGGCCACGCTCG | - | 13 |
| 3'Jun | CCATGCTGAGCGGTTGGTTGAGAGTATGATGC | *BlpI* | 14 |
| nsP4-F | GGCGGCTTCCTGTTACTCGACACGG | - | 15 |
| 5'ProGFP | ATCGATGAACGATATC*GGCCGCCGCTACACGCTATGGCG* | *EcoV* | 16 |
| 3'ProGFP | CCGGAATGCTAGCTTAAGCTCGAGATCTGAGTCCG | *NheI* | 17 |
| 3'UTR | CGAGCTTAAGCTAGCATTCCGGTATACAAATCGC | *NheI* | 18 |
| T7t | GGCTAGGTCGGCGGCCGCAAAARACCCCTCAAGACCCG | *NotI* | 19 |
| GSP1 | CCGCCGAGTCGCTCCAGTTGGCG | - | 20 |
| GSP2 | CGGGTTCTCCAGGACGTCCTTCAAG | - | 21 |
| 5'RACEseq | GGCGGCGGCATGGTCGTTGGACGACCGG | - | 22 |
| Cap-R | CCTTCAGCATAGTCATGGCCTTCTTTGG | - | 23 |
| GFP-R | TTAAGCTCGAGATCTGAGTCCGGAC | - | 24 |

| | | | |
|---|---|---|---|
| Les sites de restriction sont soulignés. Les séquences en italique font partie de la séquence de la nsP4 et les séquences indiquées en gras font partie de la séquence de la GFP. | | | |

### EXEMPLE 1 : CLONAGE DU GENOME COMPLET DU VMS

Une construction intégrale d'ADNc de VMS, pBS-VMS, a été obtenue à partir de fragments d'ADNc (numérotés 1 à 3) couvrant le génome complet de VMS, obtenus à partir de la séquence publiée précédemment (VILLOING et al., 2000 ; WESTON et al., 2002, précités ; numéro d'accès GENBANK . NC_003433.1/GI:19352423). Chaque fragment a été amplifié par transcription inverse suivie de PCR (RT-PCR) en employant l'ARN génomique de VMS comme matrice. L' ARN a été extrait à l'aide du kit de purification QIAamp Viral ARN (Qiagen), à partir des surnageants des cellules VMS infectées concentrés au PEG. Les amorces (P1 à P6) utilisées pour la transcription inverse et l'amplification PCR sont représentées dans le Tableau 1.

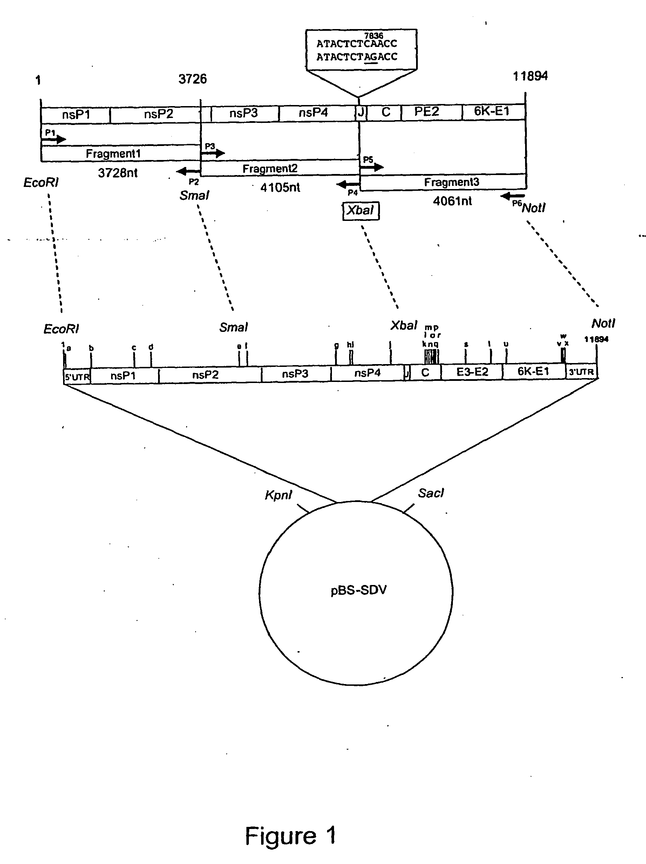

Les fragments d'ADNc obtenus ont été ligaturés les uns avec les autres et assemblés au site multiple de clonage du plasmide pBlueScript en utilisant les sites de restriction *EcoRI, SmaI, XbaI* et *NotI.* Le plasmide obtenu est représenté sur la Figure 1.

Comme indiqué sur la Figure 1, un site de restriction *XbaI* a été introduit artificiellement pour faciliter des étapes ultérieures de clonage. Le séquencage de la construction pBS-VMS a mis en évidence 42 variations par rapport à la séquence publiée. Ces variations sont listées dans le Tableau II ci-après, et indiquées par les lettres (a à x) sur la construction de pBS-VMS représentée sur la Figure 1.

Parmi elles, 8 mutations fortuites ont été corrigées comme suit : différentes portions du génome ARN de VMS correspondant aux régions du génome ADNc contenant les mutations ont été ré-amplifiées par RT-PCR. Chaque produit de PCR a été séquencé, et si sa séquence était correcte, a été inséré à la place de son homologue dans la construction pBS-VMS en utilisant les sites de restriction appropriés et la technologie standard (SAMBROOK et al., Molecular cloning: a laboratory manual, 2nd ed. Cold Spring Harbor Laboratory Press. Cold Spring Harbor, N.Y., 1989). A l'exception du site de restriction *XbaI,* la construction finale pBS-VMS contient une copie ADNc exacte du génome ARN de VMS.

**TABLEAU II**

| Position (nt) | | | Nucléotide* | Acide aminé* |
|---|---|---|---|---|
| 5'UTR | 2 | a | T? A | |
| nsP1 | 35 | b | T? A | L? Q |
| | 1123 | c | G? A | D? N |
| | 1519 | d | G? A | G? R |
| | 1531 | d | C? A | L? I |
| nsP2 | 1958 | | C? A | A? D |
| | 2345 | | G? A | G? E |
| | 2477 | | A? G | E? G |
| | 2669 | | T? C | L? P |
| | 3728 | e | G? C | R? P |
| | 3934 | f | CG? GT | R? V |
| | 3938 | f | G? T | R? L |
| | 3941 | f | C? T | S? F |
| nsP3 | 5084 | | C? T | P? L |
| | 5095 | | A? G | I? V |
| nsP4 | 6107 | g | T? C | L? P |
| | 6392 | | T? A | F? Y |
| | 6471 | h | A? C | E? D |
| | 6505 | i | A? G | K? E |
| | 7467 | j | A? C | E? D |
| jun | 7836 | | CA? AG | |
| Capsid | 8337 | k | T? A | F? I |
| | 8383 | l | T? A | V? D |
| | 8415 | m | T? A | C? S |
| | 8469 | n | G? T | G? W |
| | 8482 | o | A? C | N? T |
| | 8486 | o | T? - | Frameshift |
| | 8490 | o | G? - | |
| | 8504 | p | T? G | |
| | 8506 | p | T? C | |
| | 8510 | p | T? A | |
| | 8539 | q | G? - | |
| | 8553-55 | r | CcA? GcC | P? A |
| | 8556 | r | T? A | F? I |
| E2 | 9310 | s | C? T | T? M |
| | 9937 | t | T? G | L? W |
| 6K | 10422 | u | GCG? AGC | A? S |
| E1 | 10858 | | A? G | E? G |
| | 11709 | v | A? G | R? G |
| | 11722 | w | A? - | Frameshift |
| | 11739 | w | T? - | |
| | 11751 | x | G? - | |

| | | | | |
|---|---|---|---|---|
| * la première position correspond à la séquence publiée; la deuxième position correspond à la séquence d'ADNc déterminée dans la présente étude. Les mutations accidentelles qui ont été corrigées sont indiquées en gras. Les lettres (a-x) se réfèrent à la Figure 1 | | | | |

### EXEMPLE 2 : CONSTRUCTION D'UN ARN GENOMIQUE PERMETTANT LA SYNTHESE DES PROTEINES NON STRUCTURALES DE VMS DANS DES CELLULES DE POISSONS, ET D'UN REPLICON DE VMS EXPRIMANT LA GFP ET LA LUCIFERASE

L'insert d'ADNc du VMS a été transféré à partir de pBS-VMS dans un vecteur pcDNA3 (Stratagene) entre les sites de restriction *EcoRI* et *NotI,* en aval du promoteur précoce immédiat (IE) du cytomégalovirus (CMV) et d'un promoteur de l'ARN polyémrase T7. La construction résultante est nommée pSDV.

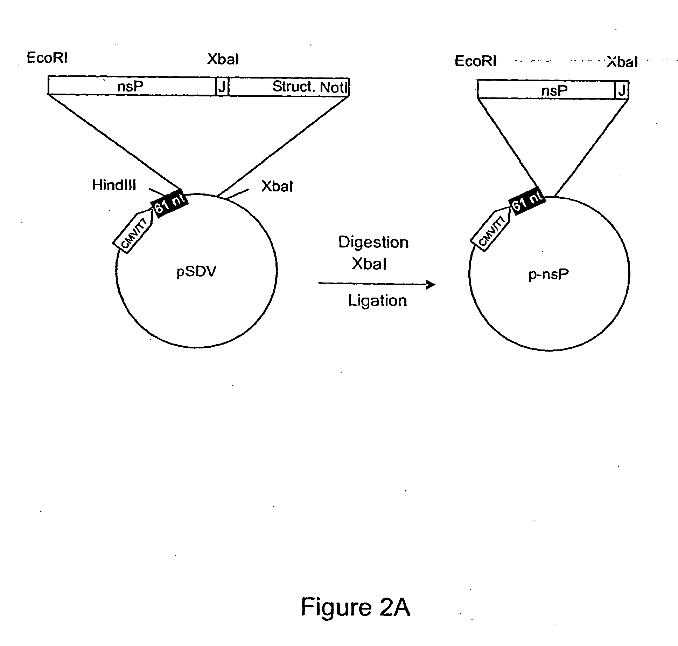

La région de l'ADNc codant pour les protéines de structure a été enlevée par digestion avec *XbaI* dont un site se trouve dans la région de jonction et l'autre dans le multisite de clonage du pcDNA3 en aval de l'ADNc. La construction a été autoligaturée pour donner le plasmide p-nsP, représenté sur la Figure 2A).

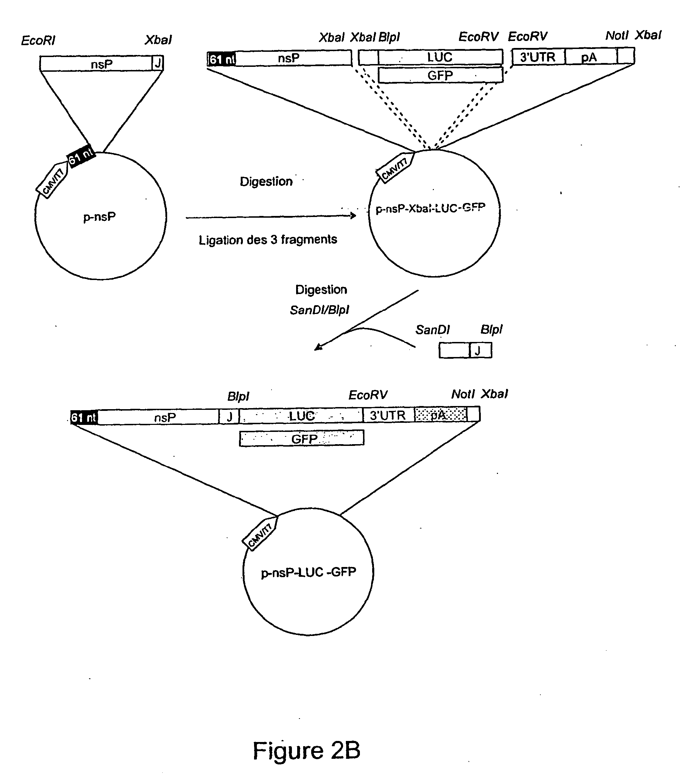

Le plasmide p-nsP a ensuite été linéarisé par *XbaI* pour insérer, en aval de la région codant pour les protéines non-structurales, une séquence codant pour la GFP ou pour la luciférase (LUC) précédée par la fin de la région de jonction et² suivie de l'extrémité 3' non traduite du VMS fusionnée à une queue poly (A). Le site *XbaI* artificiel de la région de jonction a été enlevé en interchangeant un fragment *SanDI*/*BlpI.* Le cadre de lecture de la GFP ou celui de la luciférase est encadré par deux sites uniques de restriction : un site *EcoRV* et un site *BIpI*. Dans ces constructions finales, nommées p-nsP-GFP et p-nsP-LUC, l'ensemble des prompteurs CMV/T7 est séparé de l'extrémité 5' du génome du VMS par 61 nucléotides appartenant au multisite de clonage du pcDNA3. Ces constructions sont représentées sur la Figure 2B.

Pour évaluer la fonctionnalité de ces constructions pour l'expression des gènes rapporteurs GFP et LUC, chacune d'entre elles a été utilisée pour transfecter des cellules BF-2, qui ont été incubées à 10°C, dans des puits de plaques de culture (6x10⁵ cellules/puits), et l'activité luciférase et l'activité GFP ont été mesurées quotidiennement.

Pour la mesure de l'activité luciférase, les cellules transfectées sont récoltées avant la mesure, lavées au PBS, et lysées avec 75 µl de tampon de lyse 1 x (25mM Tris-phosphate (pH 7.8), 2mM DTT, 2mM 1,2-diaminocyclohexane-N,N,N',N'- acide tétraacétique, 10% glycérol, 1% Triton X-100). Les lysats sont clarifiés par centrifugation à basse vitesse, et les protéines quantifiées par la méthode de Bradford, afin de normaliser les échantillons.

50 µl de réactif luciférase (Promega) sont ajoutés à des aliquotes des lysats clarifiés.

Dans le cas de la GFP, l'expression dans les cellules transfectées est directement suivie par observation au microscope en lumière UV.

L'expression des protéines non structurales est détectée par immunofluorescence dès le lendemain de la transfection. En revanche, quel que soit le temps après transfection, on ne détecte ni activité luciférase, ni fluorescence de la GFP.

Les résultats sont illustrés dans le tableau III ci-dessous.

**TABLEAU III**

| Construction plasmidique | Expression des protéines non structurales | Expression de la GFP | Expression de la luciférase |
|---|---|---|---|
| p-nsP-GFP | +++ | - | |
| p-nsP-LUC | +++ | | - |

Ces résultats indiquent que l'ARN viral a été transcrit, mais que l'on n'observe pas d'expression des gènes rapporteurs GFP ou luciférase, qui sont placés sous le contrôle du promoteur subgénomique 26S du VMS.

Ceci permet de supposer que le complexe réplicatif viral n'est pas fonctionnel, du fait que l'extrémité 5' ne soit pas strictement identique à celle du génome du VMS.

### Utilisation d'un ribozyme comme espaceur :

Une séquence de ribozyme en tête de marteau (séquence HH) a été fusionnée au premier nucléotide de l'extrémité 5' du génome ADNc du VMS, de la manière suivante : un fragment *HindIII* de p-nsP, contenant les 2 premiers Kb d'ADNc de VMS a été enlevé et sous-cloné dans un plasmide pUC19, pour donner la construction pUC-SDV *HindIII*. A partir de cette construction, un fragment *BamHI*/*NaeI* contenant l'extrémité 5' du génome de VMS a été enlevé, et remplacé par un fragment d'ADN synthétique généré par hybridation de deux oligonucléotides partiellement complémentaires de 79 et 80 nucléotides comprenant la séquence du ribozyme en tète de marteau fusionnée à l'extrémité 5' du génome de VMS, et remplissage à l'aide du fragment de Klenow de l'ADN polymérase de T4. La séquence de ces oligonucléotides (5'RIBO and 3'RIBO) figure dans le Tableau I.

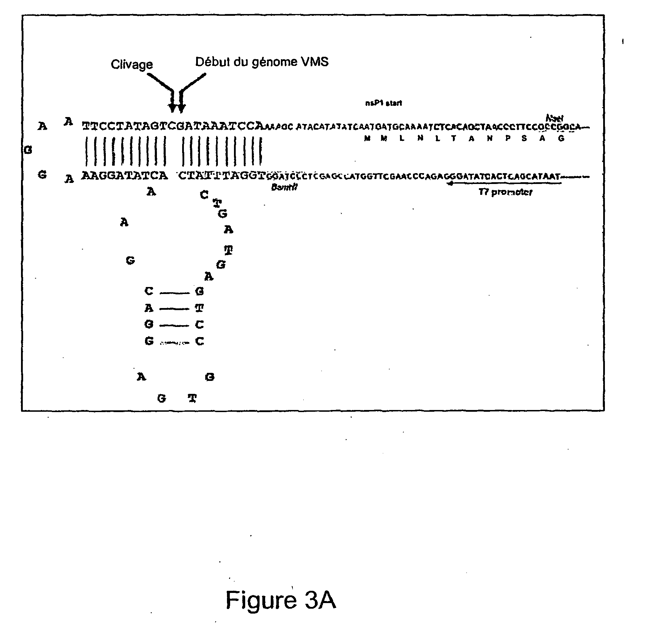

La séquence du ribozyme en tête de marteau est représentée sur la Figure 3A ; les sites *BamHI* et *NaeI* et le promoteur T7 sont soulignés. Le site de clivage du ribozyme et le début du génome du VMS sont indiqués par des flèches

Après digestion avec les enzymes de restriction appropriées, le fragment d'ADN synthétique a été inséré dans le plasmide pUC-SDV *HindIII* pour donner la construction pUC-HH-SDV *HindIII.* Le fragment *HindIII* modifié a été récupéré à partir de cette construction et réinséré dans le plasmide p-nsP, pour donner la contruction pHH-nsP.

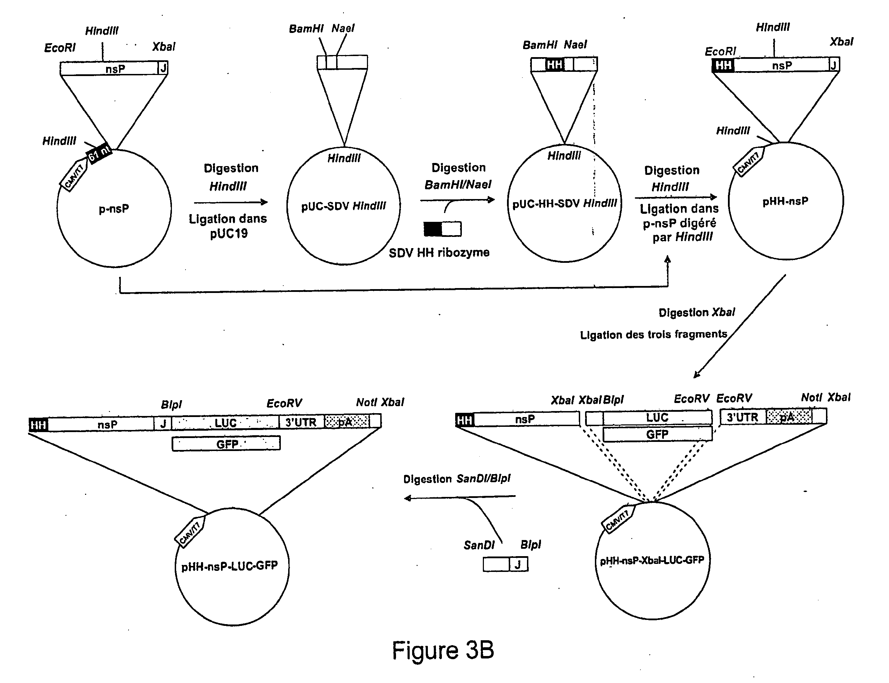

La construction pHH-nsP résultante a ensuite été linéarisée par *XbaI* et modifiée de la même façon que dans le cas de p-nsP : insertion, en aval de la région codant pour les protéines non-structurales, d'une séquence codant pour la GFP ou pour la luciférase (LUC) encadrée des sites *BlpI* et EcoRV, précédée par la fin de la région de jonction et suivie de l'extrémité 3' non traduite du VMS fusionnée à une queue poly (A), correction du site *XbaI* artificiel. Les constructions finales sont nommées pHH-nsP-GFP et pHH-nsP-LUC. Les étapes d'obtention de ces constructions sont représentées sur la Figure 3B.

La fonctionnalité de ces constructions a été évaluée de la même façon que celle des constructions p-nsP-GFP et p-nsP-LUC.

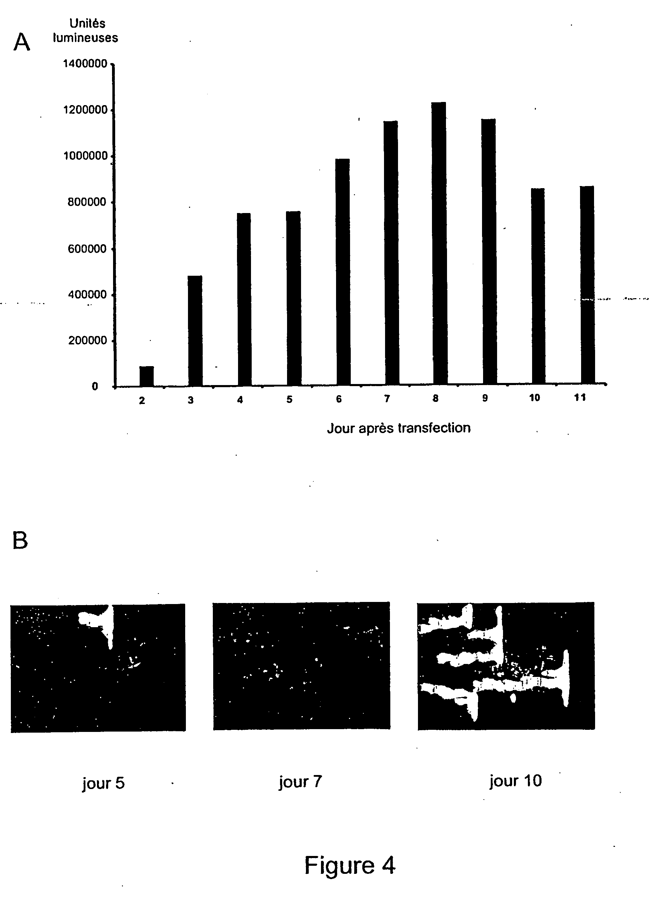

Les résultats sont illustrés par la Figure 4. Une activité luciférase significative est détectée dès 2 jours après transfection, et augmente jusqu'à 9 jours après transfection (Figure 4A). L'expression de GFP est mise en évidence 4 jours après transfection, et est optimale, comme pour la luciférase, au bout de 9 jours (Figure 4B).

Ces résultats indiquent que le complexe réplicase du VMS (nsP1, nsP2, nsP3 et nsP4) exprimé à partir des vecteurs pHH-nsP-LUC ou -GFP, est biologiquement actif, et est capable de répliquer et de transcrire un ARN subgénomique contenant les gènes rapporteurs. Ces données montrent également que le clivage à l'extrémité 5' du génome de VMS a été efficacement réalisé par le ribozyme en tête de marteau

### EXEMPLE 3: CONSTRUCTION D'UN ADNc RECOMBINANT INFECTIEUX DE VMS

Un clone d'ADNc infectieux de VMS a été construit de la manière suivante:

La région codant pour les protéines structurales de VMS a été insérée entre les sites *BIpI* et *EcoRV* de pHH-nsP-LUC, en remplacement de la séquence codant la luciférase, pour donner la construction pHH-SDV.

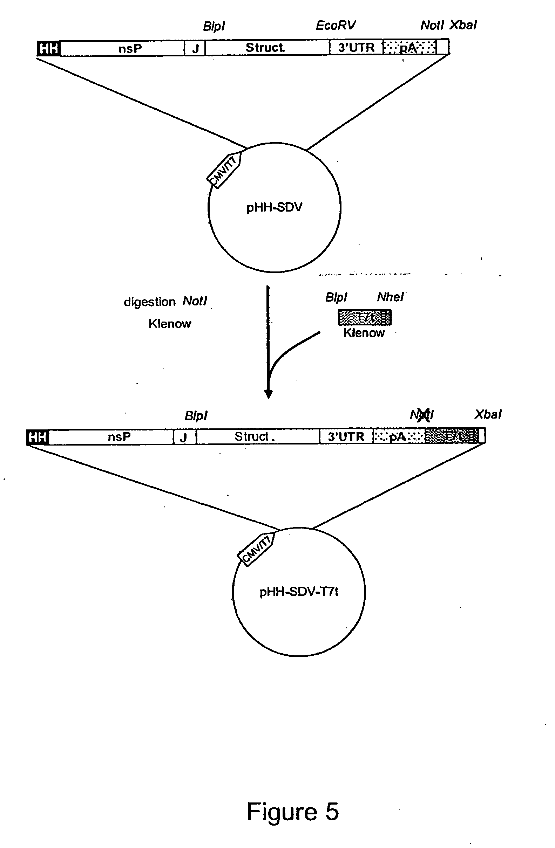

Cette construction a en outre été modifiée par insertion d'un terminateur T7 (T7t) : le vecteur pHH-SDV a été linéarisé par digestion avec l'enzyme de restriction *NotI* ; et ligaturé à bouts francs avec un fragment *BlpI*/*NheI* du vecteur pET-14b (Novagen) contenant un terminateur T7 (préalablement à la ligature, les extrémités des deux fragments ont été remplies à l'aide du fragment de Klenow de l'ADN polymérase T4). La construction résultante est dénommée pHH-SDV-T7t. Les étapes d'obtention de cette construction sont schématisées sur la Figure 5.

Cette construction a été utilisée pour transfecter des cellules BF-2 infectées par le virus de la vaccine recombinant vTF7-3, qui exprime l'ARN polymérase T7 (FUERST et al., 1986, précité). Les cellules BF-2 (environ 1.2x10⁶ cellules/puits) sont cultivées dans des plaques à 12 puits et infectées avec vTF7-3 (multiplicité d'infection =5). Après 1 heure d'adsorption à 37°C, les cellules sont lavées 2 fois, et transfectées avec 1,6µg de pSDV, en utilisant le réactif Lipofectamine 2000, selon les instructions du fabricant (Invitrogen). Les cellules sont incubées 7 heures à 37°C et lavées avec du milieu MEM avant d'être transférées à 10°C et incubées à cette température pendant 7 ou 10 jours. Dans certaines expériences, des transfections ont été effectuées en suivant le même protocole, mais sans infection préalable des cellules avec vTF7-3.

7 jours et 10 jours après transfection, les cellules sont fixées avec un mélange alcool/acétone 1/1 à 20°C pendant 15 minutes, et incubées avec un assortiment d'anticorps monoclonaux dirigés contre des protéines structurales ou non-structurales du VMS, dilués à 1/1000 en PBS-Tween. Après 45 minutes d'incubation à température ambiante, les cellules sont lavées et incubées avec un anticorps anti-immunoglobulines de souris (Biosys, France). Après lavage, les cellules sont examinées au microscope sous éclairage UV.

Parallèlement, les surnageants sont récupérés, clarifiés par centrifugation à 10,000 x g dans une microcentrifugeuse, et utilisés pour infecter des cellules BF-2 fraîches, cultivées à 10°C en monocouche dans des plaques à 24 puits. Les cellules ainsi infectées sont analysées par immunofluorescence comme décrit ci-dessus.

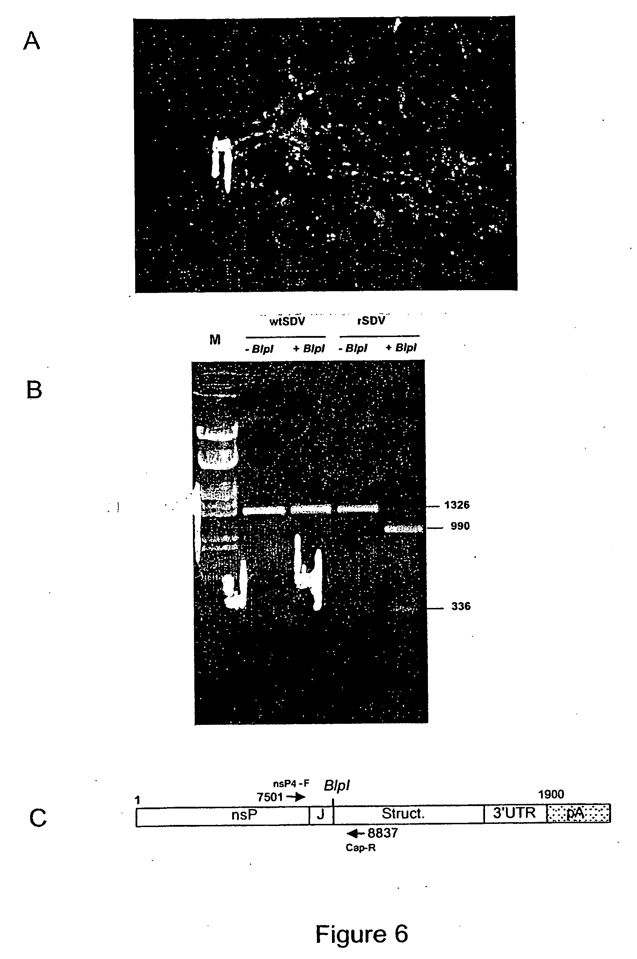

Les résultats observés 7 jours après transfection sont illustrés par la Figure 6A : quelques petits foyers apparaissent ; leur taille est plus importante 10 jours après transfection, ce qui reflète probablement une infection cellule à cellule par le VMS recombinant.

Le VMS recombinant possède un site de restriction *BlpI,* qui est absent du virus sauvage. Pour vérifier que le virus produit par les cellules infectées est bien le VMS recombinant, l'ARN est extrait des cellules infectées avec le VMS recombinant, à l'issue du premier passage, et utilisé comme matrice pour effectuer une RT-PCR en utilisant les amorces NsP4-F et CapR, encadrant le site *BlpI.* La position de ces amorces est indiquée sur la Figure 6C. Une RT-PCR est également effectuée, avec les mêmes amorces, à partir d'ARN extrait de cellules infectées avec le virus sauvage.

Les produits d'amplification sont digérés par *BlpI,* et leurs profils de restriction sont comparés. Les résultats sont illustrés par la Figure 6B. En l'absence de *BlpI* (-*BlpI*), on observe un fragment de 1326 nucléotides avec le VMS recombinant, comme avec le virus sauvage. Après digestion par *BlpI* (+*BlpI*), ce fragment demeure intact dans le cas du virus sauvage, et donne un fragment de 990 nucléotides et un fragment de 336 nucléotides dans le cas du VMS recombinant.

### EXEMPLE 4: INFECTION IN VIVO PAR LE VMS RECOMBINANT.

Afin de vérifier le pouvoir infectieux du VMS recombinant, 50 jeunes truites arc-en-ciel *(Oncorhynchus mykiss)* saines sont infectés par immersion pendant 2 heures dans un aquarium rempli d'eau à 10°C, contenant 5 x10⁴ UFP/ml de VMS sauvage ou de VMS recombinant, obtenu à partir de cellules BF-2 infectées. L'aquarium est ensuite complété à 30 litres avec de l'eau fraîche. Des poissons utilisés à titre de contrôle sont traités dans les mêmes conditions avec du milieu de culture à la place de la suspension virale.

3 semaines après infection, quelques poissons ont été sacrifiés, et des homogénats d'organes de chaque poisson ont été utilisés pour infecter des cellules BF-2. L'analyse de ces cellules par immunofluorescence, comme décrit à l'exemple 3 ci-dessus, montre la présence de virus dans les cellules infectées avec les homogénats d'organes des poissons infectés avec le VMS sauvage ou avec le VMS recombinant (résultats non montrés).

Tous les poissons sacrifiés étaient positifs pour le VMS, le titre viral étant d'approximativement 10⁷ UFP/ml pour le VMS sauvage comme pour le VMS recombinant..

### EXEMPLE 5 : CONSTRUCTION D'UN VMS RECOMBINANT INFECTIEUX EXPRIMANT UN GENE HETEROLOGUE.

Pour produire un virus recombinant infectieux exprimant la GFP, l'ADNc infectieux pHH-SDV-T7t est modifié de deux manières différentes, afin d'insérer une cassette d'expression additionnelle exprimant la GFP.

### 1) Construction de l'ADNc infectieux pH-SDV-GFPfirst

La construction pHH-nsP-GFP est utilisée comme matrice pour générer deux produits d'amplification PCR séparés: le produit « GFP PCR » est obtenu en utilisant les amorces 5'GFP et 3'GFP (Tableau 1) ; le produit « VMS subgénomique PCR » est obtenu en utilisant les amorces 5'nsP4 (7706-7750) et 3'Jun (Tableau 1). Comme la localisation exacte et la taille minimale du promoteur subgénomique du VMS n'ont pas encore été déterminées, un fragment d'environ 100 nucléotides, contenant la fin de la séquence de nsP4 et la région de jonction a été utilisé. Le promoteur subgénomique du VMS est ensuite ligaturé par PCR en 3' de la séquence codant pour la GFP, en mélangeant les deux produits issus de la première amplification et en utilisant les amorces 5'GFP et 3'Jun.

Le produit d'amplification résultant (GFP-SDVPro) est digéré par *BlpI* et inséré dans la construction pHH-SDV-T7t, préalablement digérée par *BlpI* pour obtenir l'ADNc infectieux pHH-SDV-GFPfirst.

### 2) Construction de l'ADNc infectieux pHH-SDV-GFPsecond

Dans cette construction, la cassette d'expression de la GFP est insérée en aval des gènes de structure.

Deux produits d'amplification PCR sont générés:
- le promoteur subgénomique de VMS fusionné à la GFP (produit PCR1), en utilisant comme amorces 5'ProGFP et 3'ProGFP (Tableau 1), et comme matrice la construction pHH-nsP-GFP ;
- la région 3'non-traduite de VMS fusionnée à une queue poly(A) et au terminateur T7 (produit PCR2), en utilisant comme amorces 3'UTR et T7t (Tableau 1), et comme matrice la construction pHHSDV-T7t.

Les produits PCR1 et PCR2 sont assemblés par PCR en utilisant les amorces 5'ProGFP et T7t. Le produit d'amplification PCR est digéré par *EcoRV* et *Notl* et inséré dans la construction pHH-SDV digérée par les mêmes enzymes, pour obtenir l'ADNc infectieux pHH-SDV-GFPsecond.

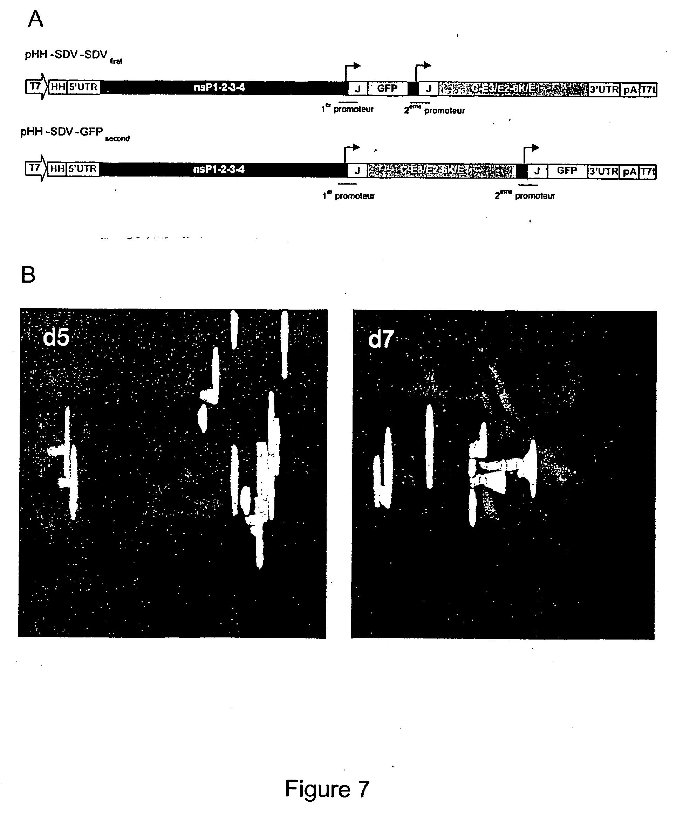

Ces deux constructions sont schématisées sur la Figure 7A.

Ces constructions sont utilisées pour transfecter des cellules BF-2 infectées par vTF7, et l'expression de GFP est suivie quotidiennement. Les résultats sont illustrés par la Figure 7B.

L'expression de la GFP est détectable à partir de 7 jours après transfection pour les deux constructions pHH-SDV-GFP. On observe toutefois une expression de la GFP plus intense lorsque le gène GFP est localisé en aval de ceux des protéines de structure dans le génome.

Pendant le clonage du gène GFP dans pSDV pour obtenir la construction pHH-SDV-GFPfirst, un plasmide (pHH-SDV-GFP₃) suspecté de contenir 3 cassettes GFP a été sélectionné.

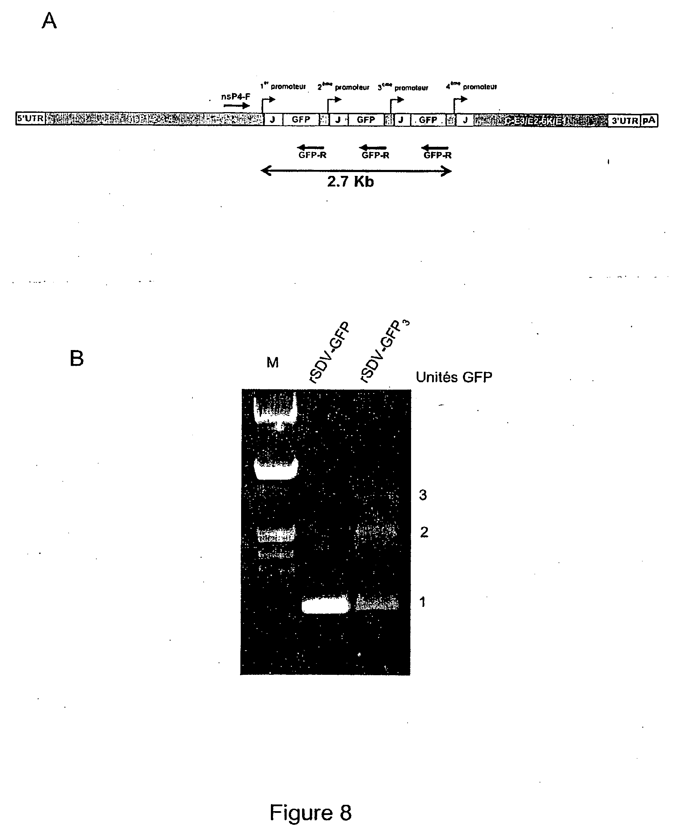

Ce plasmide est schématisé sur la Figure 8A.

Une RT-PCR utilisant les amorces nsP4-F and GFP-R (Table 1) a permis de confirmer" qu'effectivement 3 cassettes GFP étaient présentes dans le plasmide pHHSDV-GFP₃.

Les résultats de cette RT-PCR sont représentés sur la Figure 8B. Ensemble, ces trois cassettes GFP représentent un fragment d'ADN de 2.7 Kb.

Cette construction a été transfectée dans des cellules BF-2 infectées par vTF7-3, et l'apparition de foyers de cellules infectées au bout de 9 jours a confirmé la fonctionnalité de cette construction.

Ces résultats montrent que le VMS peut contenir un acide nucléique hétérologue qui est plus de 20% plus long que le génome du virus sauvage.

## Revendications

1. ADN recombinant dérivé du génome d'un alphavirus de salmonidé, comprenant :
- un promoteur de transcription, et en aval dudit promoteur et sous contrôle transcriptionnel de celui-ci ;
- une séquence d'espacement d'au moins 5 nucléotides ;
- l'ADNc de l'ARN génomique d'un alphavirus de salmonidé
**caractérisé en ce que** la séquence d'espacement est définie par la formule générale (1) ci-après :
5' X₁CTGANGARX₂B₂X'₂YGAAAX₃B₃X'₃TH 3' (I)
dans laquelle, A, T, G, et C ont leur signification usuelle ; H représente C, T, ou A ; Y représente A ou G ; R représente C ou T ; N représente A, T, G, ou C ; X₁ représente un oligonucléotide d'au moins 3 nucléotides, de préférence de 6 à 10 nucléotides, de séquence complémentaire de celle de l'extrémité 5' du génome dudit alphavirus ; X₂ représente un oligonucléotide d'au moins 3 nucléotides, de préférence de 3 à 5 nucléotides, de séquence quelconque ; B₂ représente un oligonucléotide de 4 ou 5 nucléotides, de séquence quelconque ; X'₂ représente un oligonucléotide complémentaire de X₂; X₃ représente un oligonucléotides d'au moins 2 nucléotides, de préférence de 6 à 10 nucléotides, de séquence quelconque ; B₃ représente un oligonucléotides de 4 ou 5 nucléotides, de séquence quelconque ; X'₃ représente un oligonucléotide complémentaire de X₃.

2. ADN recombinant selon la revendication 1, **caractérisé en ce que** l'insert d'ADNc d'alphavirus de salmonidé contient une ou plusieurs cassette(s) d'expression(s) dont chacune contient : un exemplaire dudit promoteur subgénomique, et, en aval dudit promoteur subgénomique et sous contrôle transcriptionnel de celui-ci, une séquence hétérologue que l'on souhaite exprimer, ou un site de clonage permettant l'insertion de cette séquence.

3. Procédé de préparation d'un réplicon d' ARN d'un alphavirus de salmonidé, **caractérisé en ce qu'**il comprend l'introduction in vitro dans une cellule-hôte, d'un ADN recombinant selon la revendication 1, et la culture de ladite cellule-hôte.

4. Réplicon d'ARN d'un alphavirus de salmonidé atténué susceptible d'être obtenu par un procédé selon la revendication 3.

5. Procédé de préparation d'un alphavirus de salmonidé recombinant, **caractérisé en ce qu'**il comprend l'introduction d'un ADN recombinant selon une quelconque des revendications 1 ou 2, ou d'un réplicon d'ARN selon la revendication 4, in vitro dans une cellule-hôte où est exprimé l'ensemble des protéines structurales dudit alphavirus nécessaires à son encapsidation, et la culture de ladite cellule-hôte.

6. Procédé selon la revendication 5, **caractérisé en ce que** l'ensemble de l'information génétique permettant l'expression desdites protéines structurales est portée par ledit ADN recombinant ou ledit réplicon d'ARN.

7. Procédé selon la revendication 5, **caractérisé en ce que** tout ou partie de l'information génétique permettant l'expression desdites protéines structurales est fournie en *trans* par la cellule hôte.

8. Alphavirus de salmonidé atténué recombinant susceptible d'être obtenu par un procédé selon une quelconque des revendication 5 à 7.

9. Utilisation d'un ADN recombinant selon une quelconque des revendications 1 à 2, d'un réplicon d'ARN d'alphavirus de salmonidé atténué selon la revendication 4, ou d'un alphavirus de salmonidé atténué recombinant selon la revendication 8 pour l'obtention d'un vaccin.

10. Vaccin comprenant un alphavirus de salmonidé atténué recombinant selon la revendication 8.

## Claims

1. Recombinant DNA derived from the genome of a Salmonidae alphavirus, and comprising:
- a transcription promoter and, downstream of said promoter and under transcriptional control thereof;
- a spacer sequence of at least 5 nucleotides;
- the cDNA of the genomic RNA of a Salmonidae alphavirus,
**characterized in that** the spacer sequence is defined by general formula (I) below:
5' X₁CTGANGARX₂B₂X'₂YGAAAX₃B₃X'₃TH 3' (I)
in which A, T, G, and C have their usual meaning; H represents C, T or A; Y represents A or G; R represents C or T; N represents A, T, G or C; X₁ represents an oligonucleotide of at least 3 nucleotides, preferably from 6 to 10 nucleotides, of sequence complementary to that of the 5' end of the genome of said alphavirus; X₂ represents an oligonucleotide of at least 3 nucleotides, preferably from 3 to 5 nucleotides, of any sequence; B₂ represents an oligonucleotide of 4 or 5 nucleotides, of any sequence; X'₂ represents an oligonucleotide complementary to X₂; X₃ represents an oligonucleotide of at least 2 nucleotides, preferably of 6 to 10 nucleotides, of any sequence; B₃ represents an oligonucleotide of 4 to 5 nucleotides, of any sequence; X'₃ represents an oligonucleotide complementary to X₃.

2. Recombinant DNA according to Claim 1, **characterized in that** the Salmonidae alphavirus cDNA insert contains one or more expression cassette(s), each of which contains: a copy of said subgenomic promoter and, downstream of said subgenomic promoter and under transcriptional control thereof, a heterologous sequence that it is desired to express, or a cloning site for the insertion of this sequence.

3. Method for preparing a Salmonidae alphavirus RNA replicon, **characterized in that** it comprises the in vitro introduction, into a host cell, of a recombinant DNA according to Claim 1, and the culturing of said host cell.

4. Attenuated Salmonidae alphavirus RNA replicon that can be obtained by means of a method according to Claim 3.

5. Method for preparing a recombinant Salmonidae alphavirus, **characterized in that** it comprises the in vitro introduction of a recombinant DNA according to either one of Claims 1 and 2, or of an RNA replicon according to Claim 4, into a host cell in which all of the structural proteins of said alphavirus that are required for its encapsidation are expressed, and the culturing of said host cell.

6. Method according to Claim 5, **characterized in that** all the genetic information for the expression of said structural proteins is carried by said recombinant DNA or said RNA replicon.

7. Method according to Claim 5, **characterized in that** all or part of the genetic information for the expression of said structural proteins is provided in *trans* by the host cell.

8. Attenuated recombinant Salmonidae alphavirus that can be obtained by means of a method according to any one of Claims 5 to 7.

9. Use of a recombinant DNA according to either one of Claims 1 and 2, of an attenuated Salmonidae alphavirus RNA replicon according to Claim 4, or of an attenuated recombinant Salmonidae alphavirus according to Claim 8, for obtaining a vaccine.

10. Vaccine comprising an attenuated recombinant Salmonidae alphavirus according to Claim 8.

## Patentansprüche

1. Rekombinante DNA, die aus dem Genom eines Salmoniden-Alphavirus stammt und Folgendes umfasst:
- einen Transkriptionspromotor und stromabwärts des Promotors und unter dessen Transkriptionskontrolle
- eine Spacer-Sequenz von mindestens 5 Nukleotiden,
- die cDNA der genomischen RNA eines Salmoniden-Alphavirus,
**dadurch gekennzeichnet, dass** die Spacer-Sequenz durch die folgende allgemeine Formel (I) definiert ist:
5' X₁CTGANGARX₂B₂X'₂YGAAAX₃B₃X'₃TH 3' (I),
in der A, T, G und C die üblichen Bedeutungen haben, H für C, T oder A steht, Y für A oder G steht, R für C oder T steht, N für A, T, G oder C steht, X₁ für ein Oligonukleotid mit mindestens 3 Nukleotiden, vorzugsweise 6 bis 10 Nukleotiden, steht, dessen Sequenz komplementär zu derjenigen des 5'-Endes des Genoms des Alphavirus ist, X₂ für ein Oligonukleotid mit mindestens 3 Nukleotiden, vorzugsweise 3 bis 5 Nukleotiden, mit beliebiger Sequenz steht, B₂ für ein Oligonukleotid mit 4 oder 5 Nukleotiden mit beliebiger Sequenz steht, X'₂ für ein zu X₂ komplementäres Oligonukleotid steht, X₃ für ein Oligonukleotid mit mindestens 2 Nukleotiden, vorzugsweise 6 bis 10 Nukleotiden, mit beliebiger Sequenz steht, B₃ für ein Oligonukleotid mit 4 oder 5 Nukleotiden mit beliebiger Sequenz steht, X'₃ für ein zu X₃ komplementäres Oligonukleotid steht.

2. Rekombinante DNA nach Anspruch 1, **dadurch gekennzeichnet, dass** das Salmoniden-Alphavirus-cDNA-Insert eine oder mehrere Expressionskassette(n) enthält, die jeweils Folgendes enthalten: ein Exemplar des subgenomischen Promotors und stromabwärts dieses subgenomischen Promotors und unter dessen Transkriptionskontrolle eine heterologe Sequenz, die exprimiert werden soll, oder eine Klonierungsstelle, die die Insertion dieser Sequenz gestattet.

3. Verfahren zur Herstellung eines RNA-Replikons eines Salmoniden-Alphavirus, **dadurch gekennzeichnet, dass** es das Einbringen einer rekombinanten DNA nach Anspruch 1 in vitro in eine Wirtszelle und das Züchten der Wirtszelle umfasst.

4. RNA-Replikon eines abgeschwächten Salmoniden-Alphavirus, das durch ein Verfahren nach Anspruch 3 erhältlich ist.

5. Verfahren zur Herstellung eines rekombinanten Salmoniden-Alphavirus, **dadurch gekennzeichnet, dass** es das Einbringen einer rekombinanten DNA nach einem der Ansprüche 1 oder 2 oder eines RNA-Replikons nach Anspruch 4 in vitro in eine Wirtszelle, in der die gesamten Strukturproteine des Alphavirus, die für dessen Einkapselung notwendig sind, exprimiert werden, und das Züchten der Wirtszelle umfasst.

6. Verfahren nach Anspruch 5, **dadurch gekennzeichnet, dass** die gesamte genetische Information, die die Expression der Strukturproteine gestattet, auf der rekombinanten DNA oder dem RNA-Replikon vorliegt.

7. Verfahren nach Anspruch 5, **dadurch gekennzeichnet, dass** die gesamte oder ein Teil der genetische(n) Information, die/der die Expression der Strukturproteine gestattet, durch die Wirtszelle in trans bereitgestellt wird.

8. Rekombinantes abgeschwächtes Salmoniden-Alphavirus, das durch ein Verfahren nach einem der Ansprüche 5 bis 7 erhältlich ist.

9. Verwendung einer rekombinanten DNA nach einem der Ansprüche 1 bis 2, eines RNA-Replikons eines abgeschwächten Salmoniden-Alphavirus nach Anspruch 4 oder eines rekombinanten abgeschwächten Salmoniden-Alphavirus nach Anspruch 8 zur Gewinnung eines Impfstoffs.

10. Impfstoff, der ein rekombinantes abgeschwächtes Salmoniden-Alphavirus nach Anspruch 8 umfasst.
